# EUROPÄISCHE PATENTANMELDUNG

(11) **EP 1 285 690 A2**
(43) Veröffentlichungstag der Anmeldung: **26.02.2003**
(21) Anmeldenummer: 02011422.9
(22) Anmeldetag: 24.05.2002
(51) Int. Cl.: B01J 2/16

(54) **Verfahren zur Herstellung von Feststoffen in Granulatform**

(30) Priorität: 08.08.2001 DE 10138927
(71) Anmelder: Glatt Ingenieurtechnik GmbH, D-99427 Weimar (DE)
(72) Erfinder: Rümpler, Karlheinz, Dr., 99425 Weimar (DE); Willert, Olaf, 99439 Wohlsborn (DE); Waskow, Mike, 07747 Jena (DE)
(74) Vertreter: Maucher, Wolfgang, Dipl.-Ing.

(57) **Zusammenfassung**

Die Erfindung betrifft ein Verfahren zur Herstellung von Feststoffen in Granulatform, in einer kontinuierlich betriebenen Wirbelschicht. Die Aufgabe der Erfindung besteht darin, ein Verfahren der gattungsgemäßen Art dahingehend weiter zu entwickeln, dass mit ihm verschiedenartige Stoffsysteme verarbeitet werden können, bei dem ein oder mehrere Endprodukte entstehen. Erfindungsgemäß wird das dadurch erreicht, dass zur Herstellung von einem oder mehreren Endprodukten bei gleichzeitiger chemischer Reaktion und Trennung der Reaktionsprodukte in der Wirbelschicht
- der Wirbelschicht mehrere verschiedene Reaktionsprodukte zugeführt werden,
- ein oder mehrere Reaktionspartner im stöchiometrischen Verhältnis in die Wirbelschicht eingedüst werden,
- durch Zuführung eines Fluidisiermittelstrom in der Wirbelschicht eine Trennung der verschiedenen Reaktionsprodukte auf der Oberfläche der sich bildenden Granulate erfolgt,
- ein Reaktionsprodukt in der Wirbelschicht ein Feststoff ist,
- die bei der chemischen Reaktion entstehenden Nebenprodukte verdampft und mit dem Fluidisiermittelstrom aus dem Prozess entfernt werden,
- die Nebenprodukte Wasser und/oder Kohlendioxid sind,
- die Reaktionstemperatur, das Gleichgewicht der chemischen Reaktion und die Granulateigenschaften über die Temperatur des Fluidisierungsmediums, die Fluidisierungsgeschwindigkeit, die Verweilzeit des Materials in der Wirbelschicht und über die zugeführte Sprühmenge eingestellt werden.

## Beschreibung

Die Erfindung betrifft ein Verfahren zur Herstellung von Feststoffen in Granulatform in einer kontinuierlich betriebenen Wirbelschicht gemäß den im Oberbegriff des Hauptanspruches genannten Merkmalen.

Wirbelschichten werden zur Herstellung von Granulaten und Agglomeraten aus feststoffhaltigen Flüssigkeiten in allen Bereichen der Industrie eingesetzt. Einsatzmöglichkeiten sind aus der chemischen und pharmazeutischen Industrie, der Lebensmittelindustrie, der keramischen Industrie, der Biotechnologie und der Abfallindustrie bekannt. Als feststoffhaltige Flüssigkeiten werden dabei Lösungen von Feststoffen, Suspensionen, Schlämme, Emulsionen und Schmelzen verwendet, die als Sprühflüssigkeit mittels Bedüsungssystem einer Wirbelschicht zugeführt werden. So ist aus der DE 27 33 935 bekannt, vorgemischte Stoffe als Flüssigkeit oder Feststoff in eine Wirbelschicht einzubringen und zu granulieren oder zu agglomerieren. Reaktionen finden dabei in der Regel in der vorgemischten flüssigen Phase statt.

Weiterhin ist aus der DE 43 29 205 und DE 196 03 849 bekannt, Natriumcarbonatlösung und Wasserstoffperoxid getrennt über eine Mehrstoffdüse in eine Wirbelschicht einzubringen und im Bereich der Verdüsung zu einem neuen Produkt reagieren zu lassen, das dann in Granulatform hergestellt wird. Nach dem in der Fachliteratur beschriebenen Bertrams-Verfahren wird Kalilauge in eine Wirbelschicht eingedüst und mit Kohlendioxid fluidisiert, so entsteht Pottasche (Kaliumcarbonat).

Die Aufgabe der Erfindung besteht darin, ein Verfahren der gattungsgemäßen Art dahingehend weiter zu entwickeln, dass mit ihm verschiedenartige Stoffsysteme verarbeitet werden können, bei dem ein oder mehrere Endprodukte entstehen.

Die Aufgabe wird erfindungsgemäß mit den Merkmalen des Anspruches 1 gelöst. Vorteilhafte Ausgestaltungen des Verfahrens sind in den Unteransprüchen angegeben.
Durch die erfindungsgemäße Lösung werden auch problematische Stoffsysteme miteinander in Reaktion gebracht, bei der ein oder mehrere Endprodukte entstehen. Dabei kommt es durch Aufdüsung einer Flüssigkeit auf den Feststoff einer Wirbelschicht schon zu einer chemischen Reaktion. Aber auch flüssige Reaktionsparameter können getrennt oder gemeinsam verdüst in der Wirbelschicht neue chemische Verbindungen hervorbringen. Die Reaktionspartner werden zum Beispiel über Mehrkomponenten-Düsen in die Wirbelschicht eingedüst. Die chemische Reaktion läuft teils im Sprühkegel der Düse und 10 teils auf der Oberfläche der fluidisierten Feststoffpartikel ab. Dabei finden gleichzeitig eine Aufbaugranulation des Produktes und eine Verdampfung der Nebenprodukte statt. Mögliche Lösungsmittel der Edukte werden ebenfalls entfernt.
Das an sich bekannte Wirbelschichtverfahren wird gekoppelt mit an sich bekannten Ein-, Zwei- oder Mehrstoffdüsen und liefert die Möglichkeit, bisher nicht auf diese Art und Weise hergestellte Granulate entstehen zu lassen. Kernstück ist immer eine chemische Reaktion mit stoff-veränderlichen Eigenschaften.

Die Wirbelschicht wird dazu benutzt, die verschiedenen Reaktionsprodukte voneinander zu trennen. Dabei muss mindestens eines der Reaktionsprodukte unter den vorliegenden Prozessbedingungen ein Feststoff sein. Durch das schalenartige Wachsen der Granulate wird erreicht, unerwünschte Nebenprodukte bis zu 100% aus dem Hauptprodukt zu entfernen. Es erfolgt eine Trennung der Reaktionsprodukte auf der Oberfläche der sich bildenden Granulate.

Bei diesem Verfahren wird durch einen aufwärts gerichteten Fluidisiermittelstrom, wie zum Beispiel Luft, Stickstoff oder ähnliches, eine Feststoffwirbelschicht aus festem Reaktionsprodukt ausgebildet. In der Wirbelschicht werden über spezielle Düsen, zum Beispiel Mehrstoffdüsen, die Reaktionspartner im stöchiometrischen Verhältnis eingedüst. Außerhalb der Düse kommt es zum Kontakt zwischen den Rohstoffen und die chemische Reaktion findet statt. Das feste Reaktionsprodukt baut sich dabei auf den in der Wirbelschicht fluidisierten Teilchen auf. Als Nebenprodukte entstehende Flüssigkeiten werden verdampft und mit dem Fluidisiermittelstrom abtransportiert. Ebenso werden auch entstehende gasförmige Nebenprodukte aus dem Prozess entfernt. Die bei exothermen Reaktionen frei werdende Reaktionswärme wird zur Verdampfung ausgenutzt. Bei endothermen Reaktionen wird die benötigte Energie durch das Fluidisiermedium zugeführt. Über die Temperatureinstellung des Fluidisierungsmediums, die Fluidisiergeschwindigkeit, die Verweilzeit sowie die Sprühmenge werden die Reaktionstemperatur, das Gleichgewicht der chemischen Reaktion und die Granulateigenschaften beeinflusst. Eine weitere Einflussmöglichkeit besteht darin, Fluidisiermittel mit katalytischen Eigenschaften zu nutzen. Die chemische Reaktion kann auch zwischen einem in der Wirbelschicht vorhandenen Feststoff und eingedüster Flüssigkeit stattfinden.

Die Erfindung wird nachfolgend in Ausführungsbeispielen näher erläutert.

### 1. Kontinuierliche Neutralisation

In einer runden Wirbelschichtanlage AGT 400 werden über eine Mehrstoffdüse zwei anorganische Flüssigkeiten (Lauge und Säure) mit dazugehöriger Luft eingedüst. Es entsteht im Sprühnebel der Düse ein anorganisches Salz, das vorhandene und freiwerdende Wasser verdampft, es wird mit dem Fluidisierungsgas weggeführt und es bilden sich Granulate.
Die Flüssigkeiten können organischer Natur sein. Eine Komponente kann organisch, die andere anorganisch sein.

### 1.1. Schwefelsäure und Monoethanolamin

In einer runden Wirbelschichtanlage AGT 400 werden über eine Mehrstoffdüse konzentrierte Schwefelsäure und Monoethanolamin mit dazugehöriger Druckluft eingedüst. Es entsteht im Sprühnebel der Düse ein Salz (Monoethanolamin-Sulfat). Anschließend findet auf der Oberfläche der sich bildenden Granulate eine Veresterung zu Aminoethylhydrogensulfat statt. Das abgespaltene Wasser wird unter den Prozessbedingungen (ca. 130 - 150°C Wirbelschichttemperatur) verdampft und mit dem Fluidisiermedium (ca. 1000 - 1200 kg/h) wegtransportiert. Die bei der Reaktion frei werdende Reaktionswärme wird zum Verdampfen des Wassers benutzt.

Die entstehenden Granulate werden durch einen sichtenden Austrag (Klassierluft ca. 100 -250 kg/h) aus der Wirbelschicht ausgetragen.

### 1.2. Monochloressigsäure und Natronlauge

In einer runden Wirbelschichtanlage AGT 400 werden über eine Mehrstoffdüse Monochloressigsäure und Natronlauge mit dazugehöriger Druckluft eingedüst. Es entsteht im Sprühnebel der Düse ein Salz (Natrium-Monochloracetat). Als Nebenprodukt der Reaktion entsteht Wasser. Das Wasser wird unter den Prozessbedingungen (ca. 70 - 80°C Wirbelschichttemperatur) verdampft und wird mit dem Fluidisiermedium (ca. 800 - 1000 kg/h) wegtransportiert. Die bei der Reaktion frei werdende Reaktionswärme wird zum Verdampfen des Wassers benutzt.

Die entstehenden Granulate werden durch einen sichtenden Austrag (Klassierluft ca. 80 - 150 kg/h) aus der Wirbelschicht ausgetragen.

### 2. Chemische Reaktion mit Freisetzung von Gas

In einer rechteckigen Wirbelschichtanlage GFG 20 wird als feinkörniger Feststoff ein Erdalkalicarbonat fluidisiert und mit einer anorganischen Säure über ein Düsensystem bedüst. Es kommt in der Wirbelschicht zur chemischen Reaktion. Das entstehende Kohlendioxid wird mit dem Fluidisierungsgas weggeführt.
Die Säure kann auch organischer Natur sein.
Der verwendete Feststoff war feinkörnig und wurde im Wirbelbett bei einer Leerrohrgeschwindigkeit von 2m/s fluidisiert. Die verwendete Säure kann konzentriert oder wässrig sein. Die Zulufttemperatur wurde in Abhängigkeit der zu verdampfenden Wassermenge zwischen 100 und 300°C eingestellt. Das entweichende Kohlendioxid und der entstehende Wasserdampf gehen in das Abgas über.

### 3. Chemische Reaktionen mit Oxiden

In einer rechteckigen Wirbelschichtanlage GFG 20 wird als feinkörniger Feststoff ein Metalloxid fluidisiert und mit einer organischen Säure über ein Düsensystem bedüst. Es kommt in der Wirbelschicht zur chemischen Reaktion. Das entstehende Wasser wird verdampft und mit dem Fluidisierungsgas weggeführt.
Die Säure kann auch anorganischer Natur sein.
Der verwendete Feststoff war feinkörnig und wurde im Wirbelbett bei einer Leerrohrgeschwindigkeit von 2m/s fluidisiert. Die verwendete Säure kann konzentriert oder wässrig sein. Die Zulufttemperatur wurde in Abhängigkeit der zu verdampfenden Wassermenge zwischen 100 und 300°C eingestellt. Das entweichende Kohlendioxid und der entstehende Wasserdampf gehen in das Abgas über.

### 4. Oxidation

In einer runden Wirbelschichtanlage AGT 400 wird im Chargenbetrieb ein Metallpulver fluidisiert. Im Fall von Kupferpulver war die Fluidisierungsgeschwindigkeit im Leerrohr 1 m/s. Durch die Anwesenheit von Sauerstoff in der Fluidisierungsluft wird das Kupferpulver (allgemein Metallpulver) oxidiert. Im Fall von Kupferpulver mussten in der Wirbelschicht Temperaturen zwischen 400 und 500°C eingestellt werden. Das Aufheizen der Wirbelschicht erfolgte von Umgehungstemperatur bis auf ca. 200°C mit Gradienten von 150 K/h, danach bis auf Endtemperatur von ca. 450°C mit Gradienten von 50 K/h.
Danach wird das sich gebildete Kupferoxidpulver mit Umgebungsluft bis auf ca. 60°C abgekühlt.

## Patentansprüche

1. Verfahren zur Herstellung von Feststoffen in Granulatform, in einer kontinuierlich betriebenen Wirbelschicht, bei dem die Ausgangsstoffe reine Flüssigkeiten, Lösungen, Suspensionen, Schmelzen, Feststoffe oder Gase sind und der Wirbelschicht von unten ein Fluidisierungsmittelstrom zugeführt wird und ein klassierender Austrag des Endproduktes erfolgt, **dadurch gekennzeichnet, dass** zur Herstellung von einem oder mehreren Endprodukten bei gleichzeitiger chemischer Reaktion und Trennung der Reaktionsprodukte in der Wirbelschicht
- der Wirbelschicht mehrere verschiedene Reaktionsprodukte zugeführt werden,
- ein oder mehrere Reaktionspartner im stöchiometrischen Verhältnis in die Wirbelschicht eingedüst werden,
- durch Zuführung eines Fluidisiermittelstrom in der Wirbelschicht eine Trennung der verschiedenen Reaktionsprodukte auf der Oberfläche der sich bildenden Granulate erfolgt,
- ein Reaktionsprodukt in der Wirbelschicht ein Feststoff ist,
- die bei der chemischen Reaktion entstehenden Nebenprodukte verdampft und mit dem Fluidisiermittelstrom aus dem Prozess entfernt werden,
- die Nebenprodukte Wasser und/oder Kohlendioxid sind,
- die Reaktionstemperatur, das Gleichgewicht der chemischen Reaktion und die Granulateigenschaften über die Temperatur des Fluidisierungsmediums, die Fluidisierungsgeschwindigkeit, die Verweilzeit des Materials in der Wirbelschicht und über die zugeführte Sprühmenge eingestellt werden.

2. Verfahren nach Anspruch 1, **dadurch gekennzeichnet, dass** zur Herstellung eines Salzes in Granulatform über eine Mehrstoffdüse eine flüssige Lauge und eine flüssige Säure mit Luft in die Wirbelschicht eingedüst werden.

3. Verfahren nach Anspruch 1 oder 2, **dadurch gekennzeichnet, dass** die eingedüsten Flüssigkeiten organischer, anorganischer oder anorganischer und organischer Natur sind.

4. Verfahren nach Anspruch 1, **dadurch gekennzeichnet, dass** zur Herstellung von Monoethanolamin-Sulfat über eine Mehrstoffdüse konzentrierte Schwefelsäure und Monoethanolamin mit Luft in die Wirbelschicht eingedüst werden, wobei die Temperatur der Wirbelschicht ca. 130 bis 150°C beträgt.

5. Verfahren nach Anspruch 1, **dadurch gekennzeichnet, dass** zur Herstellung von Natrium-Monochloracetat über eine Mehrstoffdüse Monochloressigsäure und Natronlauge mit Luft in die Wirbelschicht eingedüst werden, wobei die Temperatur der Wirbelschicht ca. 70 bis 80°C beträgt.

6. Verfahren nach Anspruch 1, **dadurch gekennzeichnet, dass** ein Erdalkalicarbonat in der Wirbelschicht bei Leerrohrgeschwindigkeiten der Fluidisierungsluft von ca. 2 m/s und einer Zulufttemperatur von ca. 100 bis 300°C fluidisiert und über ein Düsensystem mit einer anorganischen oder organischen Säure besprüht wird.

7. Verfahren nach Anspruch 1, **dadurch gekennzeichnet, dass** ein Metalloxid in der Wirbelschicht bei Leerrohrgeschwindigkeiten der Fluidisierungsluft von ca. 2 m/s und einer Zulufttemperatur von ca. 100 bis 300°C fluidisiert und über ein Düsensystem mit einer anorganischen oder organischen Säure besprüht wird.

8. Verfahren nach Anspruch 1, **dadurch gekennzeichnet, dass** ein Metallpulver in der Wirbelschicht bei Leerrohrgeschwindigkeiten der Fluidisierungsluft von ca. 1 m/s fluidisiert wird, wobei das Metallpulver in der Wirbelschicht von Umgebungstemperatur bis auf ca. 200°C mit Gradienten von ca. 150 K/h und mit Gradienten von ca. 50 K/h auf eine Endtemperatur von ca. 400 bis 500°C aufgeheizt wird und danach mit Umgebungsluft auf ca. 60°C abgekühlt wird.
